# EUROPEAN PATENT APPLICATION

(11) **EP 2 036 576 A1**
(43) Date of publication of application: **18.03.2009**
(21) Application number: 07018213.4
(22) Date of filing: 17.09.2007
(51) Int. Cl.: A61K 49/00, G01N 33/574

(54) **ED-B fibronectin as stratification marker for antitumor drugs**

(71) Applicant: Bayer Schering Pharma Aktiengesellschaft, 13353 Berlin (DE)
(72) Inventor: Berndorff, Dietmar, 13467 Berlin (DE); Dinkelborg, Ludger, 13465 Berlin (DE)

(57) **Abstract**

The present invention relates to A method for stratifying tumor cells comprising the steps:(a) providing a sample comprising tumor cells to be analysed, (b) contacting said sample with an ED-B fibronectin-specific molecule and assessing the binding thereof to tumor cells in said sample, and (c) stratifying said tumor cells based on the assessment of step (b).

## Description

The present invention refers to the use of molecues for the extra-domain B (ED-B) of fibronectin, for example of labeled antibodies or antibody fragments against the ED-B domain as diagnostic reagents for stratifying a tumor with regard to its sensitivity to anti-tumor drugs and particularly as stratification marker for EGF/EGFR antagonists.

Inhibitors of the epidermal growth factor receptor (EGFR) are used in tumor therapy, particularly for the treatment of patients with non-small-cell lung carcinoma (NSCLC). The sensitivity of EGFR-containing human NSCLC cell lines to EGFR inhibition is dependent on the degree to which they have undergone an epithelial to mesenchymal transition (EMT). NSCLC cell lines which express the epithelial cell junction protein E-cadherin show a greater sensitivity to EGFR inhibition compared to cell lines having undergone EMT (Thomson et al., Cancer Res. 65 (2005), 9455-9462).

Further, it was found that the clinical activity of EGFR antagonists in NSCLC patients may be predicted by determination of EMT in human tumor cells (Yauch et al., Clin. Cancer Res. 11 (2005), 8686-8698).

EMT is characterized by loss of epithelial markers, e.g. E-cadherin, and gain of mesenchymal markers, e.g. vimentin. EMT leads to de-differentiation, loss of adhesive constraint and enhanced mutility and invasion. These properties are hallmarks of increased malignancy. Thus, EMT provides a mechanism for carcinoma cells to acquire this more aggressive phenotype (Lee et al., J. Cell. Biol. 172 (2006), 973-981; Christiansen and Rajasekaran, Cancer Res. 66 (2006), 8319-8326). Detection of EMT by routine analytic methods is, however, difficult, because this process probably occurs transiently in discrete areas within tumors (Gotzman et al., Mutation Res.566 (2004)9-20).

Thus, it was an object of the present invention to provide a reliable and sensitive method which allows determination of epithelial versus mesenchymal phenotype in human tumor cells or tissues or other cells or tissues of interest.

In a first aspect the present invention refers to a method for stratifying a tumor cell or tumor tissue comprising the steps of
(a) contacting an ED-B fibronectin-specific molecule with a tumor cell or a tumor tissue and assessing the binding of the ED-B fibronectin-specific molecule to the tumor cell or the tumor tissue, and
(b) stratifying said tumor cell or tumor tissue based on the assessment of step (a).

In a further aspect the present invention refers to a method of detecting, particularly of imaging, epithelial mesenchymal transition (EMT) in a cell or tissue, particularly in a tumor cell or tumor tissue, comprising the steps of
(a) contacting an ED-B fibronectin-specific molecule with a cell or a tissue and assessing the binding of the ED-B fibronectin-specific molecule to the cell or tissue, and
(b) identifying said cell or tissue as epithelial-type cell or tissue or as mesenchymal-type cell or tissue based on the assessment of step (a).

Still a further aspect of the present invention refers to the use of an ED-B fibronectin-specific molecule for the manufacture of an agent for stratifying a tumor cell or tumor tissue as being sensitive or refractory against treatment.

Still a further aspect of the present invention refers to the use of an ED-B fibronectin binding molecule for the manufacture of an agent for detecting epithelial mesenchymal transition.

Still a further aspect of the present invention refers to a composition or kit for stratifying a tumor cell or tumor tissue comprising an ED-B fibronectin-specific binding molecule conjugated with a detectable labelling group.

Still a further aspect of the present invention refers to a composition or kit for detecting epithelial mesenchymal transition comprising an ED-B fibronectin-specific binding molecule conjugated with a detectable labelling group.

According to the present invention it was surprisingly found that determination of ED-B fibronectin can be used for the selection or stratification of a tumor cell or tumor tissue and/or for the detection of EMT. Binding of tumor cells or tumor tissue to ED-B specific molecules indicates sensitivity for anti-tumor drugs, such as EGF/EGFR antagonists and/or epithelial phenotype. Lack of binding or reduced binding of tumor cells or tumor tissue to ED-B specific molecules indicates resistance against anti-tumor drugs, such as EGF/EGFR antagonists and/or mesenchymal phenotype. Thus, the present invention is particularly suitable for the selection or stratification of tumor patients and their sensitivity towards anti-tumor treatment, such as treatment with EGF/EGFR antagonists.

Molecules specific for the ED-B domains of fibronectin, a sequence of 91 amino acids, which is inserted by alternative splicing into the fibronectin molecule (Castellani et al. (1994), Int. J. Cancer 59, 612-18), are already described in WO 97/45544, WO 01/62800, WO 03/055917, WO 03/07649 and WO 2005/0223318, which are herein incorporated by reference. Preferred binding molecules are molecules that bind directly and specifically to the ED-B domains, such as, for example, antibodies against the ED-B domains or fragments of such antibodies, for example antibody fragments that can be obtained by proteolytic cleavage, e.g. Fab-, Fab'- F(ab₂) fragments etc., or recombinant antibody fragments, e.g. single-chain Fv-fragments. The ED-B-binding molecules are preferably used as conjugates with labeling groups that are suitable for diagnostic applications.

A preferred embodiment of the invention relates to the use of antibody L19 or fragments of this antibody (L19 derivatives), which are present as conjugates with labeling groups.

L19 is the scFv fragment (scFv: single chain variable antibody fragment) of a monoclonal antibody against the extra-domain B (ED-B) of fibronectin and has the following amino acid sequence (SEQ ID NO.1):

| (VH): | | |
|---|---|---|
| EVQLLESGGG | LVQPGGSLRL | SCAASGFTFS |
| SFSMSWVRQA | PGKGLEWVSS | ISGSSGTTYY |
| ADSVKGRFTI | SRDNSKNTLY | LQMNSLRAED |
| TAVYYCAKPF | PYFDYWGQGT | LVTVSS |
| | | |
| (Linker): | | |
| GDGSSGGSGG | ASTG | |
| | | |

| (VL): | | |
|---|---|---|
| EIVLTQSPGT | LSLSPGERAT | LSCRASQSVS |
| SSFLAWYQQK | PGQAPRLLIY | YASSRATGIP |
| DRFSGSGSGT | DFTLTISRLE | PEDFAVYYCQ |
| QTGRIPPTFG | QGTKVEIK | |

Especially preferred are L19 derivatives comprising
(aa) at least one antigen binding site for the extra-domain B (ED-B) of fibronectin comprising the complementarity-determining regions HCDR3 and/or LCDR3, shown in Table 1, or a variant thereof, which exhibits a deletion, insertion and/or substitution of up to 5 amino acids in the HCDR3 region and up to 6 amino acids in the LCDR3 region, whereby the antigen binding site exhibits the same function as the native L19 shown in SEQ ID NO. 1,
(ab) at least one antigen binding site for the extra-domain B (ED-B) of fibronectin comprising the complementarity-determining regions HCDR1, HCDR2, HCDR3, LCDR1, LCDR2 and LCDR3, shown in Table 1, or a variant thereof, which exhibits a deletion, insertion and/or substitution of up to 3 amino acids in the HCDR1 region, of up to 8 amino acids in the HCDR2 region, of up to 5 amino acids in the HCDR3 region, of up to 6 amino acids in the LCDR1 region, of up to 4 amino acids in the LCDR2 region and of up to 6 amino acids in the LCDR3 region, whereby the antigen binding site exhibits the same function as the native L19 shown in SEQ ID NO.1, or
(ac) at least one antigen binding site for the extra-domain B (ED-B) of fibronectin comprising the sequence of the native L19, shown in SEQ ID NO. 1, or a variation thereof, which exhibits a deletion, insertion and/or substitution of up to 30 amino acids, whereby the antigen binding site exhibits the same function as the native L19 shown in SEQ ID NO. 1, and optionally
(ba) an amino acid sequence Xaa₁-Xaa₂-Xaa₃-Cys (SEQ ID NO. 2), whereby Xaa₁, Xaa₂, and Xaa₃, independently of one another, represent any naturally occurring amino acid,
(bb) an amino acid sequence Xaa₁-Xaa₂-Xaa₃-Cys-Xaa₄ (SEQ ID NO. 3), whereby Xaa₁, Xaa₂, Xaa₃, and Xaa₄, independently of one another, represent any naturally occurring amino acid,
(bc) an amino acid sequence (His)ₙ (SEQ ID NO. 4), whereby n is an integer from 4 to 6, or
(bd) an amino acid sequence that comprises the sequence shown in SEQ ID NO. 5, SEQ ID NO. 6 or SEQ ID NO. 7,
whereby the C-terminus of (aa), (ab), or (ac) is optionally bonded via a peptide bond to the N-terminus of (ba), (bb), (bc) or (bd).

Within the scope of this invention, the labeled L19 derivative comprises an N-terminal antigen binding site for the extra-domain B (ED-B) of fibronectin selected from the antigen binding sites (aa), (ab) or (ac) and optionally a C-terminal amino acid sequence selected from the amino acid sequences (ba), (bb), (bc) or (bd), whereby the antigen binding site exhibits the same function as the native L19 shown in SEQ ID NO. 1.

According to this invention, the antigen binding sites for the extra-domain B (ED-B) of fibronectin of the labeled L19 derivative (aa) or (ab) comprise the complementarity-determining regions HCDR3 and/or LCDR3 or HCDR1, HCDR2, HCDR3, LCDR1, LCDR2 and LCDR3, shown in Table 1. Within the scope of this invention, the complementarity-determining regions HCDR1, HCDR2, HCDR3, LCDR1, LCDR2 and LCDR3 are defined as follows:

**Table 1**

| **Region⁽¹⁾** | **CDR Length⁽²⁾ (in Amino Acids)** | **Sequence** | **Maximum (Preferred) Variations** |
|---|---|---|---|
| HCDR1 | 5 | S F S M S (SEQ ID NO. 8) | 3 (2, 1) |
| HCDR2 | 17 | S I S G S S G T T Y Y A D S V K G (SEQ ID NO. 9) | 8 (7,6,5,4,3,2,1) |
| HCDR3 | 7 | P F P Y F D Y (SEQ ID NO. 10) | 5 (4,3,2,1) |
| LCDR1 | 12 | R A S Q S V S S S F L A (SEQ ID NO. 11) | 6 (5,4,3,2,1) |
| LCDR2 | 7 | YASSRA(SEQ ID NO. 12) | 4 (3,2,1) |
| LCDR3 | 10 | C Q Q T G R I P P T (SEQ ID NO.13) | 6 (5,4,3,2,1) |

| | | | |
|---|---|---|---|
| ⁽¹⁾HCDRx: Complementarity-determining region x the heavy antibody chain; LCDRx: complementarity-determining region x the light antibody chain. ⁽²⁾CDR length: Length of the complementarity-determining region. | | | |

In addition to the complementarity-determining regions defined in Table 1, the antigen binding sites for the extra-domain B (ED-B) of fibronectin of the labeled L19 derivative (aa) or (ab) can also comprise variants of these regions. According to the invention, a variant of the HCDR1 region comprises a deletion, insertion and/or substitution of up to 3 amino acids in the HCDR1 region, i.e., a deletion, insertion and/or substitution of 1, 2 or 3 amino acids relative to the sequence (SEQ ID NO. 8) shown in Table 1. A variant of the HCDR2 region comprises a deletion, insertion and/or substitution of up to 8 amino acids in the HCDR2 region, i.e., a deletion, insertion and/or substitution of 1, 2, 3, 4, 5, 6, 7 or 8 amino acids relative to the sequence (SEQ ID NO. 9) shown in Table 1. Moreover, a variant of the HDCR3 region comprises a deletion, insertion and/or substitution of up to 5 amino acids in the HCDR3 region, i.e., a deletion, insertion and/or substitution of 1, 2, 3, 4 or 5 amino acids relative to the sequence (SEQ ID NO. 10) shown in Table 1. A variant of the LCDR1 region, however, comprises a deletion, insertion and/or substitution of up to 6 amino acids in the LCDR1 region, i.e., a deletion, insertion and/or substitution of 1, 2, 3, 4, 5 or 6 amino acids relative to the sequence (SEQ ID NO. 11) shown in Table 1. In addition, a variant of the LCDR2 region comprises a deletion, insertion and/or substitution of up to 4 amino acids in the LCDR2 region, i.e., a deletion, insertion and/or substitution of 1, 2, 3 or 4 amino acids relative to the sequence (SEQ ID NO. 12) shown in Table 1. A variant of the LCDR3 region comprises a deletion, insertion and/or substitution of up to 6 amino acids in the LCDR3 region, i.e., a deletion, insertion and/or substitution of 1, 2, 3, 4, 5 or 6 amino acids relative to the sequence (SEQ ID NO. 13) shown in Table 1.

According to this invention, the antigen binding site for the extra-domain B (ED-B) of fibronectin of the labeled L19 derivative (ac) comprises the sequence of native L19, shown in SEQ ID NO. 1, or a variation thereof, which exhibits a deletion, insertion and/or substitution of up to 30 amino acids, i.e., a deletion, insertion and/or substitution of 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids relative to the sequence shown in SEQ ID NO. 1.

The amino acid sequences (ba), (bb) or (bc) of the labeled L19 derivative comprise the sequences Xaa₁-Xaa₂-Xaa₃-Cys (SEQ ID NO. 2), Xaa₁-Xaa₂-Xaa₃-Cys-Xaa₄ (SEQ ID NO. 3) or (HIS)ₙ (SEQ ID NO. 4).

In a preferred embodiment of this invention, the amino acid sequence (ba) Xaa₁-Xaa₂-Xaa₃-Cys (SEQ ID NO. 2) is the sequence Gly-Gly-Gly-Cys (SEQ ID NO. 14) or Gly-Cys-Gly-Cys (SEQ ID NO. 15). Especially preferred is the sequence Gly-Gly-Gly-Cys (SEQ ID NO. 14).

In another preferred embodiment of this invention, the amino acid sequence (bb) Xaa₁-Xaa₂-Xaa₃-Cys-Xaa₄ (SEQ ID NO. 3) is the sequence Gly-Gly-Gly-Cys-Ala (SEQ ID NO. 16) or Gly-Cys-Gly-Cys-Ala (SEQ ID NO. 17). Especially preferred is the sequence Gly-Gly-Gly-Cys-Ala (SEQ ID NO. 16).

In another preferred embodiment of this invention, the amino acid sequence (bc) (His)ₙ (SEQ ID NO. 4) is the sequence (His)₆ with n equal to 6 (SEQ ID NO. 18).

In another preferred embodiment of this invention, the N-terminus of (aa), (ab) or (ac) is optionally connected via a peptide bond to the C-terminus of a linker amino acid sequence. The linker amino acid sequence preferably has a length of up to 30 amino acids, preferably up to 25 amino acids, and especially preferably up to 22 amino acids. Especially preferred is the linker amino acid sequence, which is the sequence shown in SEQ ID NO. 19.

According to this invention, especially preferred labeled L19 derivatives comprise the sequences shown in SEQ ID NO. 1 (native L19), SEQ ID NO. 20 (AP38), SEQ ID NO. 21 (AP39), SEQ ID NO. 22 (L19-GlycysGlyCys), SEQ ID NO. 23 (L19-GlyCysGlyCysAla), SEQ ID NO. 24 (ZK225293), SEQ ID NO. 25 (ZK217691/217695), SEQ ID NO. 26 (ZK210917) and SEQ ID NO. 27 (ZK248219/248220). Especially preferred is AP39.

The binding molecule for the ED-B domain is preferably present in the form of a conjugate with a labelling group. As labelling groups, all labelling substances that are suitable for diagnostic applications, especially diagnostic applications *in vivo,* may be used, for example radiolabelling substances for radioactive detection, e.g. radioactive imaging methods, or for non-radioactive detecting, e.g. non-radioactive imaging methods, such as labelling substances that are suitable for optical imaging or magnetic resonance imaging. In an especially preferred embodiment, the labelling group is a labelling group suitable for SPECT (Single Photon Emission Computed Tomography). In a further especially preferred embodiment, the labelling group is a labelling group suitable for PET (Positron Emission Tomography).

Processes for introducing labeling substances in polypeptides, peptides and especially scFv fragments are well known in the prior art. The binding molecule is preferably labeled with a radioisotope, e.g., a radioisotope of iodine (I), indium (In), Yttrium (Y), technetium (Tc), rhenium (Re), gallium (Ga), bromine (Br) or fluorine (F). Especially preferred are the radioisotopes ¹²⁵I, ¹²³I, ¹²⁴I, ¹³¹I, ¹¹¹In, ¹⁸⁶Re, ¹⁸⁸Re, ⁸⁶Y, ^{94m}Tc, ^{99m}Tc, ⁶⁷Ga , ⁶⁸Ga, ⁷⁶Br or ¹⁸F.

In a preferred embodiment of this invention, an antibody fragment, e.g., an L19 derivative in reduced form, is used. Within the scope of this invention, the term "reduced form" means that the fragment is present in monomeric form and not, for example, in dimeric or multimeric form that is mediated by intermolecular disulfide bridges. The reduced form of the antibody fragment is preferably obtained by adding a suitable reducing agent. Suitable reducing agents are well known in the prior art and comprise TCEP (tris(2-carboxyethyl)phosphine) and 1,4-dimercapto-2,3-butanediols.

In addition, this invention provides that the pharmaceutical composition or kit, in addition to the binding molecule, optionally contains physiologically compatible adjuvants, vehicles and/or diluents. Suitable adjuvants, vehicles and/or diluents are best known to one skilled in the art in the field of pharmaceutical chemistry.

In a preferred embodiment of the present invention tumors are stratified as epithelial-type tumors if substantial binding of the ED-B fibronectin-specific molecule is observed or as mesenchymal-type tumors, if no substantial binding of the ED-B fibronectin-specific molecule is observed. In this context the term "substantial binding" comprises an immunological reaction with a target antigen which can be distinguished over unspecific binding to control cells or control tissue or binding to mesenchymal-type cells. In this context it should be noted that the term "substantial binding" also includes cellular uptake.

The cells or tissues to be analysed according to the present invention are preferably cells which are capable of undergoing EMT, or tissues comprising cells which are capable of undergoing EMT. More preferably, the cells or tissues are tumor cells or tumor tissues, e.g. lung-cancer cells or tissues, such as NSCLC cells or tissues or cells or tissues from prostate, breast, pancreas, colon, head-and-neck, ovarian, renal or cervix cancer and other tumor cells or tissues, e.g. as described by Christiansen and Rajasekaran (supra).

Preferably, the cells or tissues are from a human tumor patient. It should be noted that the method of the present invention may be carried out *in vitro* or *in vivo.* An *in vitro* diagnostic method comprises the extracorporeal testing of a cell or tissue sample, e.g. a tumor cell or tumor tissue sample, which is preferably derived from a human patient. The *in vivo* method comprises administering the ED-B fibronectin specific molecule to a subject, e.g. a human patient or an experimental animal and carrying out a determination, preferably an imaging procedure, in the subject.

The *in vivo* method of the present invention is preferably carried out by injecting the pharmaceutical composition, which comprises the ED-B-binding molecule, into a vein and/or artery of a patient to be examined and detecting the labeled ED-B-binding molecule that is bound to cells or tissue, e.g. tumor cells or tumor tissue, if present. If a radioisotope-labeled binding molecule is used, the detection can be carried out by scintigraphy, SPECT or PET.

The method of the present invention is particularly suitable for the stratification of patients, preferably tumor patients with regard to the sensitivity toward treatment, preferably toward anti-tumor treatment. The method may be carried out before the start of an anti-tumor treatment of the patient, in order to determine sensitivity toward certain treatment protocols. Alternatively or additionally, the method may also be carried out during anti-tumor treatment of the patient. The anti-tumor treatment peferably comprises administration of an EGF/EGFR antagonist, optionally in combination with the administration of further anti-tumor agents and/or radiation therapy. The EGF/EGFR antagonist may be selected from small molecule EGFR inhibitors, e.g. EGFR kinase inhibitors such as erlotinib or gefitinib. Alternatively, the EGF/EGFR antagonsist may be selected from anti-EGFR antibodies, such as cetuximab.

In an especially preferred embodiment the method of the present invention is carried out prior to the start of anti-tumor treatment using an EGFR antagonist, such as erlotinib. Thereby therapy-resistant patients may be distinguished from therapy-sensitive patients, thereby reducing ineffective tumor therapies to a high degree.

Further, the present invention shall be explained in more detail by the following examples.

### Example 1: Production of L19 Derivatives

The production of L19 derivative AP39 is carried out as described in WO 03/055917, to whose content reference is made herein.

### Example 2: Labeling of L19 Derivatives with the Aid of Radioisotopes

The production of the labeled L19 derivative AP39-Tc99m is carried out as described in WO 03/055917, to whose content reference is made herein.

### Example 3: Study of the Binding of Labeled L19 Derivatives to NSCLC Cell Lines

Binding studies were performed in two human NSCLC tumor models in mice: NCI-H292 which is an erlotinib-sensitive epithelial-type model, and Calu-6 which is an erlotinib-resistant mesenchymal-type model.

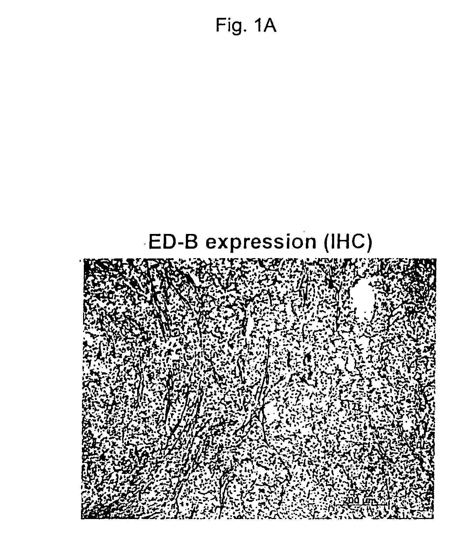

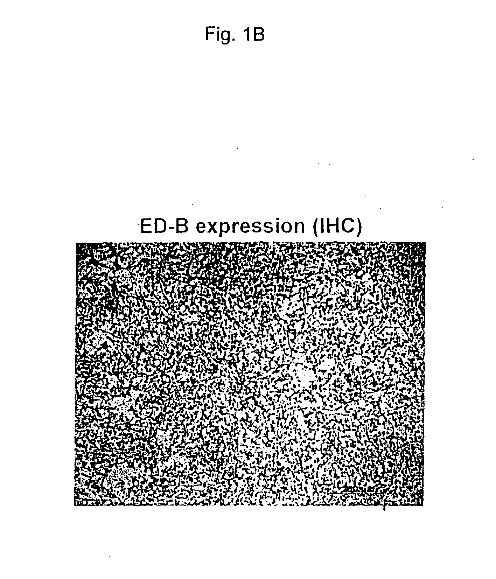

TCEP-reduced AP39 was biotinylated using 3-(N-maleimidopropionyl) biocytin (Sigma). The in vitro binding of biotinylated AP39 to frozen sections (5-10 µm) of NCI-H292 and Calu-6 tumor tissue was determined by utilizing ExtrAvidin (Sigma) and Liquid DAB Substrate Pack (BioGenex) according to the protocols of the manufacturers. A more extensive binding of biotinylated AP39 was observed in NCI-H292 (Fig. 1A) compared to Calu-6 (Fig. 1 B) tumor tissue.

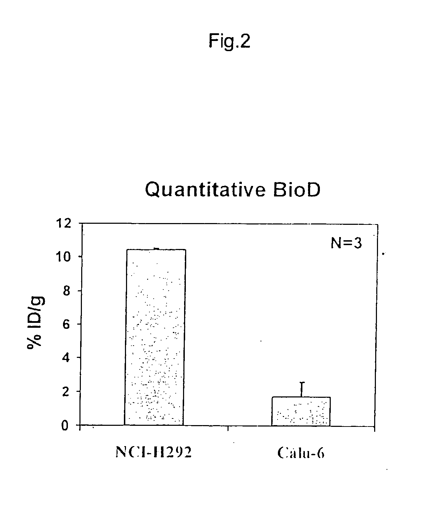

Tc-99m radiolabelled AP39 was injected intravenously in a dose of about 111 kBq into NCI-H292 and Calu-6 tumor bearing nude mice (bodyweight about 30 g). The radioactivity concentration in the tumors was measured ex vivo using a gamma-counter at 1 hour after administration of the substance. It can be gathered that the concentration of Tc-99m-AP39 is substantially higher in NCI-H292 tumor tissue (Fig. 2).

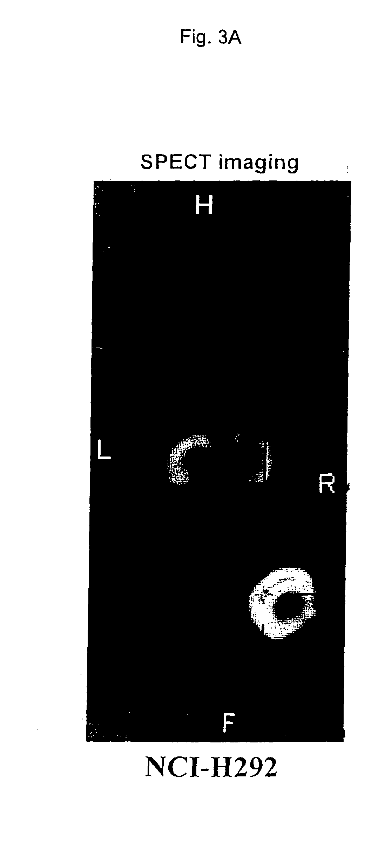

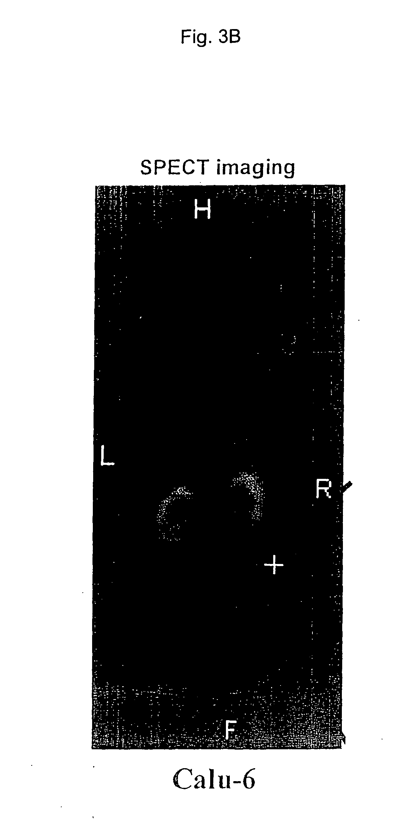

Tc-99m radiolabelled AP39 was injected intravenously in a dose of about 150 MBq into NCI-H292 and Calu-6 tumor bearing nude mice (bodyweight about 30 g). SPECT imaging was carried out at 4 hours after administration of the substance. The result of this investigation shows that the NCI-H292 (Fig. 3A) tumor can be much better depicted by SPECT imaging than the Calu-6 (Fig. 3B) tumor.

## Claims

1. A method for stratifying a tumor cell or tumor tissue comprising the steps of
(a) contacting an ED-B fibronectin-specific molecule with a tumor cell or a tumor tissue and assessing the binding of the ED-B fibronectin-specific molecule to the tumor cell or the tumor tissue, and
(b) stratifying said tumor cell or tumor tissue based on the assessment of step (a).

2. The method according to claim 1, wherein a tumor cell or tumor tissue is stratified as epithelial-type tumor cell or tissue if substantial binding of said ED-B fibronectin-specific molecule is observed or as mesenchymal-type tumor cell or tissue if no substantial binding of said ED-B fibronectin-specific molecule is observed.

3. The method of claim 1 or 2, wherein the tumor cell or tissue is selected from lung cancer, particularly NSCLC, or from prostate, breast, pancreas, colon, head-and-neck, ovarian, renal or cervix cancer.

4. The method of any one of claims 1-3, wherein the tumor cell or tumor tissue is derived from or present in a human tumor patient.

5. The method of claim 4, which is carried out before the start of an anti-tumor treatment of said patient.

6. The method of claim 4, which is carried out during the anti-tumor treatment of said patient.

7. The method of claim 5 or 6, wherein said anti-tumor treatment comprises administration of an EGF/EGFR antagonist optionally in combination with the administration of further anti-tumor agents and/or radiation therapy.

8. The method of claim 7, wherein said EGF/EGFR antagonist is selected from small molecule EGFR inhibitors, particularly erlotinib or gefitinib.

9. The method of claim 7, wherein said EGF/EGFR antagonist is selected from anti EGFR antibodies, particularly cetuximab.

10. The method of any one of claims 1-9, wherein said ED-B fibronectin-specific molecule is a peptide or an antibody.

11. The method of claim 10, wherein said ED-B fibronectin-specific molecule is an antibody having the CDR sequences from antibody L19.

12. The method of claim 11, wherein said ED-B fibronectin-specific molecule is the antibody AP39.

13. The method of any one of claims 1-12, wherein said ED-B fibronectin-specific molecule is conjugated with a detectable labelling group, particularly with an imaging group.

14. The method of claim 13, wherein the labelling group is a radioactive labelling group.

15. The method of claim 13 or 14, wherein the labelling group is a SPECT labelling group, particularly ^{99m}Tc.

16. The method of claim 14, wherein the labelling group is a PET labelling group, particularly ¹⁸F.

17. The method of any one of claims 1-16, which is carried out in vitro.

18. The method of any one of claims 1-16, which is carried out in vivo.

19. A method of detecting epithelial mesenchymal transition (EMT) in a cell or tissue, particularly in a tumor cell or tumor tissue, comprising the steps of
(a) contacting an ED-B fibronectin-specific molecule with a cell or a tissue and assessing the binding of the ED-B fibronectin-specific molecule to the cell or tissue, and
(b) identifying said cell or tissue as epithelial-type cell or tissue or as mesenchymal-type cell or tissue based on the assessment of step (a).

20. The method of claim 1 or 19, wherein the assessment in step (a) is based on an imaging procedure.

21. Use of an ED-B fibronectin-specific molecule for the manufacture of an agent for stratifying a tumor cell or a tumor tissue as being sensitive or refractory against treatment.

22. The use of claim 21, wherein the tumor cell or tumor tissue is selected from lung cancer, particularly NSCLC, or from prostate, breast, pancreas, colon, head-and-neck, ovarian, renal or cervix cancer.

23. The use of claim 21 or 22, wherein said anti-tumor treatment comprises administration of an EGF/EGFR antagonist optionally in combination with the administration of further anti-tumor agents and/or radiation therapy.

24. Use of an ED-B fibronectin binding molecule for the manufacture of an agent for detecting epithelial mesenchymal transition.

25. A composition or kit for stratifying a tumor cell or a tumor tissue comprising an ED-B fibronectin-specific binding molecule conjugated with a detectable labelling group.

26. A composition or kit for detecting epithelial mesenchymal transition comprising an ED-B fibronectin-specific binding molecule conjugated with a detectable labelling group.

27. The composition or kit of claim 25 or 26, wherein the detectable labelling group is a radioactive labelling group, particularly a SPECT labelling group or a PET labelling group.


SEQUENCE LISTING

<110> Schering Aktiengesellschaft

<120> ED-B fibronectin as stratification marker for anti-tumor drugs

<130> 41328PEP_US

<140>
<141>

<150> 07018213.4 / EP07018213
<151>

<160> 28

<170> PatentIn Ver. 2.1

<210> 1
<211> 238
<212> PRT
<213> Artificial Sequence

<220>
<223> L19 or ZK212667 = single chain variable antibody
      fragment of a monocolonal antibody against the
      extra domain B of fibronectin

<220>

<220>
<223> Description of Artificial Sequence:antibody or
      antibody fragment

<400> 1
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg

180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys
225                 230                 235

<210> 2
<211> 4
<212> PRT
<213> Artificial Sequence

<220>
<223> Description of Artificial Sequence:antibody or
      antibody fragment

<220>
<223> amino acid sequence (ba)

<400> 2
Xaa Xaa Xaa Cys
  1

<210> 3
<211> 5
<212> PRT
<213> Artificial Sequence

<220>
<223> Description of Artificial Sequence:antibody or
      antibody fragment

<220>
<223> amino acid sequence (bb)

<400> 3
Xaa Xaa Xaa Cys Xaa
  1               5

<210> 4
<211> 4
<212> PRT
<213> Artificial Sequence

<220>
<223> Description of Artificial Sequence:antibody or
      antibody fragment

<220>
<223> amino acid sequence(bc)

<400> 4
His His His His
  1

<210> 5
<211> 17

<212> PRT
<213> Artificial Sequence

<220>
<223> Description of Artificial Sequence:antibody or
      antibody fragment

<220>
<223> amino acid sequence (bd)

<400> 5
Ala Ala Ala Asp Asp Asp Ser Asp Asp Asp Tyr Lys Asp Asp Asp Asp
  1               5                  10                  15

Lys

<210> 6
<211> 23
<212> PRT
<213> Artificial Sequence

<220>
<223> Description of Artificial Sequence:antibody or
      antibody fragment

<220>
<223> amino acid sequence (bd)

<400> 6
Ala Ala Ala Asp Asp Asp Ser Asp Asp Asp Tyr Lys Asp Asp Asp Asp
  1               5                  10                  15

Lys His His His His His His
             20

<210> 7
<211> 121
<212> PRT
<213> Artificial Sequence

<220>
<223> Description of Artificial Sequence:antibody or
      antibody fragment

<220>
<223> amino acid sequence (bd)

<400> 7
Ser Gly Gly Ser Gly Gly Pro Arg Ala Ala Pro Glu Val Tyr Ala Phe
  1               5                  10                  15

Ala Thr Pro Glu Trp Pro Gly Ser Arg Asp Lys Arg Thr Leu Ala Cys
             20                  25                  30

Leu Ile Gln Asn Phe Met Pro Glu Asp Ile Ser Val Gln Trp Leu His
         35                  40                  45

Asn Glu Val Gln Leu Pro Asp Ala Arg His Ser Thr Thr Gln Pro Arg
     50                  55                  60

Lys Thr Lys Gly Ser Gly Phe Phe Val Phe Ser Arg Leu Glu Val Thr
 65                  70                  75                  80

Arg Ala Glu Trp Glu Gln Lys Asp Glu Phe Ile Cys Arg Ala Val His

85                  90                  95

Glu Ala Ala Ser Pro Ser Gln Thr Val Gln Arg Ala Val Ser Val Asn
            100                 105                 110

Pro Glu Ser Ser Arg Arg Gly Gly Cys
        115                 120

<210> 8
<211> 5
<212> PRT
<213> Artificial Sequence

<220>
<223> Description of Artificial Sequence:antibody or
      antibody fragment

<220>
<223> HCDR1 = complementarity determining region of the
      region X of the heavy antibody chain

<400> 8
Ser Phe Ser Met Ser
  1               5

<210> 9
<211> 17
<212> PRT
<213> Artificial Sequence

<220>
<223> Description of Artificial Sequence:antibody or
      antibody fragment

<220>
<223> HCDR 2 = complementarity determining region of the
      region X of the heavy antibody chain

<400> 9
Ser Ile Ser Gly Ser Ser Gly Thr Thr Tyr Tyr Ala Asp Ser Val Lys
  1               5                  10                  15

Gly

<210> 10
<211> 7
<212> PRT
<213> Artificial Sequence

<220>
<223> Description of Artificial Sequence:antibody or
      antibody fragment

<220>
<223> HCDR 3 = complementarity determining region of the
      region X of the heavy antibody chain

<400> 10
Pro Phe Pro Tyr Phe Asp Tyr
  1               5

<210> 11
<211> 12
<212> PRT
<213> Artificial Sequence

<220>
<223> Description of Artificial Sequence:antibody or
      antibody fragment

<220>
<223> LCDR 1 = complementarity determining region of the
      region X of the light antibody chain

<400> 11
Arg Ala Ser Gln Ser Val Ser Ser Ser Phe Leu Ala
 1               5                   10

<210> 12
<211> 6
<212> PRT
<213> Artificial Sequence

<220>
<223> Description of Artificial Sequence:antibody or
      antibody fragment

<220>
<223> LCDR 2 = complementarity determining region of the
      region X of the light antibody chain

<400> 12
Tyr Ala Ser Ser Arg Ala
 1               5

<210> 13
<211> 10
<212> PRT
<213> Artificial Sequence

<220>
<223> Description of Artificial Sequence:antibody or
      antibody fragment

<220>
<223> LCDR 3 = complementarity determining region of the
      region X of the light antibody chain

<400> 13
Cys Gln Gln Thr Gly Arg Ile Pro Pro Thr
 1               5                   10

<210> 14
<211> 4
<212> PRT
<213> Artificial Sequence

<220>
<223> Description of Artificial Sequence:antibody or
      antibody fragment

<220>
<223> preferred amino acid sequence (ba)

<400> 14
Gly Gly Gly Cys
  1

<210> 15
<211> 4
<212> PRT
<213> Artificial Sequence

<220>
<223> Description of Artificial Sequence:antibody or
      antibody fragment

<220>
<223> preferred amino acid sequence (ba)

<400> 15
Gly Cys Gly Cys
  1

<210> 16
<211> 5
<212> PRT
<213> Artificial Sequence

<220>
<223> Description of Artificial Sequence:antibody or
      antibody fragment

<220>
<223> preferred amino acid sequence (bb)

<400> 16
Gly Gly Gly Cys Ala
  1               5

<210> 17
<211> 5
<212> PRT
<213> Artificial Sequence

<220>
<223> Description of Artificial Sequence:antibody or
      antibody fragment

<220>
<223> preferred amino acid sequence (bb)

<400> 17
Gly Cys Gly Cys Ala
  1               5

<210> 18
<211> 6
<212> PRT
<213> Artificial Sequence

<220>
<223> Description of Artificial Sequence:antibody or
      antibody fragment

<220>
<223> preferred amino acid sequence (bc)

<400> 18
His His His His His His
1               5



<210> 19
<211> 22
<212> PRT
<213> Artificial Sequence

<220>
<223> Description of Artificial Sequence:antibody or
      antibody fragment

<220>
<223> linker amino acid sequence

<400> 19
Met Lys Tyr Leu Leu Pro Thr Ala Ala Ala Gly Leu Leu Leu Leu Ala
1               5                   10                  15

Ala Gln Pro Ala Met Ala
            20



<210> 20
<211> 242
<212> PRT
<213> Artificial Sequence

<220>
<223> Description of Artificial Sequence:antibody or
      antibody fragment

<220>
<223> AP38: labelled derivate of L19

<400> 20
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser

130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Cys

<210> 21
<211> 243
<212> PRT
<213> Artificial Sequence

<220>
<223> Description of Artificial Sequence:antibody or
      antibody fragment

<220>
<223> AP39 or ZK217052/217053: labelled derivate of L19

<400> 21
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg

165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Cys Ala

<210> 22
<211> 242
<212> PRT
<213> Artificial Sequence

<220>
<223> Description of Artificial Sequence:antibody or
      antibody fragment

<220>
<223> labelled derivate of L19

<400> 22
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg

195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Cys
225                 230                 235                 240

Gly Cys

<210> 23
<211> 243
<212> PRT
<213> Artificial Sequence

<220>
<223> Description of Artificial Sequence:antibody or
      antibody fragment

<220>
<223> labelled derivate of L19

<400> 23
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Cys

225                 230                 235                 240

Gly Cys Ala

<210> 24
<211> 283
<212> PRT
<213> Artificial Sequence

<220>
<223> Description of Artificial Sequence:antibody or
      antibody fragment

<220>
<223> ZK225293: labelled derivate of L19

<400> 24
Met Lys Tyr Leu Leu Pro Thr Ala Ala Ala Gly Leu Leu Leu Leu Ala
  1               5                  10                  15

Ala Gln Pro Ala Met Ala Glu Val Gln Leu Leu Glu Ser Gly Gly Gly
             20                  25                  30

Leu Val Gln Pro Gly Gly Ser Leu Arg Leu Ser Cys Ala Ala Ser Gly
         35                  40                  45

Phe Thr Phe Ser Ser Phe Ser Met Ser Trp Val Arg Gln Ala Pro Gly
     50                  55                  60

Lys Gly Leu Glu Trp Val Ser Ser Ile Ser Gly Ser Ser Gly Thr Thr
 65                  70                  75                  80

Tyr Tyr Ala Asp Ser Val Lys Gly Arg Phe Thr Ile Ser Arg Asp Asn
                 85                  90                  95

Ser Lys Asn Thr Leu Tyr Leu Gln Met Asn Ser Leu Arg Ala Glu Asp
            100                 105                 110

Thr Ala Val Tyr Tyr Cys Ala Lys Pro Phe Pro Tyr Phe Asp Tyr Trp
        115                 120                 125

Gly Gln Gly Thr Leu Val Thr Val Ser Ser Gly Asp Gly Ser Ser Gly
    130                 135                 140

Gly Ser Gly Gly Ala Ser Thr Gly Glu Ile Val Leu Thr Gln Ser Pro
145                 150                 155                 160

Gly Thr Leu Ser Leu Ser Pro Gly Glu Arg Ala Thr Leu Ser Cys Arg
                165                 170                 175

Ala Ser Gln Ser Val Ser Ser Ser Phe Leu Ala Trp Tyr Gln Gln Lys
            180                 185                 190

Pro Gly Gln Ala Pro Arg Leu Leu Ile Tyr Tyr Ala Ser Ser Arg Ala
        195                 200                 205

Thr Gly Ile Pro Asp Arg Phe Ser Gly Ser Gly Ser Gly Thr Asp Phe
    210                 215                 220

Thr Leu Thr Ile Ser Arg Leu Glu Pro Glu Asp Phe Ala Val Tyr Tyr
225                 230                 235                 240

Cys Gln Gln Thr Gly Arg Ile Pro Pro Thr Phe Gly Gln Gly Thr Lys
                245                 250                 255

Val Glu Ile Lys Ala Ala Ala Asp Asp Asp Ser Asp Asp Asp Tyr Lys

260                 265                 270

Asp Asp Asp Asp Lys His His His His His His
        275                 280

<210> 25
<211> 365
<212> PRT
<213> Artificial Sequence

<220>
<223> Description of Artificial Sequence:antibody or
      antibody fragment

<220>
<223> ZK217691/217695: labelled derivate of L19

<400> 25
Met Xaa Asp Thr Pro Ala Glu Val Gln Leu Leu Glu Ser Gly Gly Gly
  1               5                  10                  15

Leu Val Gln Pro Gly Gly Ser Leu Arg Leu Ser Cys Ala Ala Ser Gly
             20                  25                  30

Phe Thr Phe Ser Ser Phe Ser Met Ser Trp Val Arg Gln Ala Pro Gly
         35                  40                  45

Lys Gly Leu Glu Trp Val Ser Ser Ile Ser Gly Ser Ser Gly Thr Thr
     50                  55                  60

Tyr Tyr Ala Asp Ser Val Lys Gly Arg Phe Thr Ile Ser Arg Asp Asn
 65                  70                  75                  80

Ser Lys Asn Thr Leu Tyr Leu Gln Met Asn Ser Leu Arg Ala Glu Asp
                 85                  90                  95

Thr Ala Val Tyr Tyr Cys Ala Lys Pro Phe Pro Tyr Phe Asp Tyr Trp
            100                 105                 110

Gly Gln Gly Thr Leu Val Thr Val Ser Ser Gly Asp Gly Ser Ser Gly
        115                 120                 125

Gly Ser Gly Gly Ala Ser Thr Gly Glu Ile Val Leu Thr Gln Ser Pro
    130                 135                 140

Gly Thr Leu Ser Leu Ser Pro Gly Glu Arg Ala Thr Leu Ser Cys Arg
145                 150                 155                 160

Ala Ser Gln Ser Val Ser Ser Ser Phe Leu Ala Trp Tyr Gln Gln Lys
                165                 170                 175

Pro Gly Gln Ala Pro Arg Leu Leu Ile Tyr Tyr Ala Ser Ser Arg Ala
            180                 185                 190

Thr Gly Ile Pro Asp Arg Phe Ser Gly Ser Gly Ser Gly Thr Asp Phe
        195                 200                 205

Thr Leu Thr Ile Ser Arg Leu Glu Pro Glu Asp Phe Ala Val Tyr Tyr
    210                 215                 220

Cys Gln Gln Thr Gly Arg Ile Pro Pro Thr Phe Gly Gln Gly Thr Lys
225                 230                 235                 240

Val Glu Ile Lys Ser Gly Gly Ser Gly Gly Pro Arg Ala Ala Pro Glu
                245                 250                 255

Val Tyr Ala Phe Ala Thr Pro Glu Trp Pro Gly Ser Arg Asp Lys Arg

260                 265                 270

Thr Leu Ala Cys Leu Ile Gln Asn Phe Met Pro Glu Asp Ile Ser Val
        275                 280                 285

Gln Trp Leu His Asn Glu Val Gln Leu Pro Asp Ala Arg His Ser Thr
    290                 295                 300

Thr Gln Pro Arg Lys Thr Lys Gly Ser Gly Phe Phe Val Phe Ser Arg
305                 310                 315                 320

Leu Glu Val Thr Arg Ala Glu Trp Glu Gln Lys Asp Glu Phe Ile Cys
                325                 330                 335

Arg Ala Val His Glu Ala Ala Ser Pro Ser Gln Thr Val Gln Arg Ala
            340                 345                 350

Val Ser Val Asn Pro Glu Ser Ser Arg Arg Gly Gly Cys
        355                 360                 365

<210> 26
<211> 244
<212> PRT
<213> Artificial Sequence

<220>
<223> Description of Artificial Sequence:antibody or
      antibody fragment

<220>
<223> ZK210917: labelled derivate of L19

<400> 26
Met Xaa Asp Thr Pro Ala Glu Val Gln Leu Leu Glu Ser Gly Gly Gly
  1               5                  10                  15

Leu Val Gln Pro Gly Gly Ser Leu Arg Leu Ser Cys Ala Ala Ser Gly
             20                  25                  30

Phe Thr Phe Ser Ser Phe Ser Met Ser Trp Val Arg Gln Ala Pro Gly
         35                  40                  45

Lys Gly Leu Glu Trp Val Ser Ser Ile Ser Gly Ser Ser Gly Thr Thr
     50                  55                  60

Tyr Tyr Ala Asp Ser Val Lys Gly Arg Phe Thr Ile Ser Arg Asp Asn
 65                  70                  75                  80

Ser Lys Asn Thr Leu Tyr Leu Gln Met Asn Ser Leu Arg Ala Glu Asp
                 85                  90                  95

Thr Ala Val Tyr Tyr Cys Ala Lys Pro Phe Pro Tyr Phe Asp Tyr Trp
            100                 105                 110

Gly Gln Gly Thr Leu Val Thr Val Ser Ser Gly Asp Gly Ser Ser Gly
        115                 120                 125

Gly Ser Gly Gly Ala Ser Thr Gly Glu Ile Val Leu Thr Gln Ser Pro
    130                 135                 140

Gly Thr Leu Ser Leu Ser Pro Gly Glu Arg Ala Thr Leu Ser Cys Arg
145                 150                 155                 160

Ala Ser Gln Ser Val Ser Ser Ser Phe Leu Ala Trp Tyr Gln Gln Lys
                165                 170                 175

Pro Gly Gln Ala Pro Arg Leu Leu Ile Tyr Tyr Ala Ser Ser Arg Ala

180                 185                 190

Thr Gly Ile Pro Asp Arg Phe Ser Gly Ser Gly Ser Gly Thr Asp Phe
        195                 200                 205

Thr Leu Thr Ile Ser Arg Leu Glu Pro Glu Asp Phe Ala Val Tyr Tyr
    210                 215                 220

Cys Gln Gln Thr Gly Arg Ile Pro Pro Thr Phe Gly Gln Gly Thr Lys
225                 230                 235                 240

Val Glu Ile Lys

<210> 27
<211> 243
<212> PRT
<213> Artificial Sequence

<220>
<223> Description of Artificial Sequence:antibody or
      antibody fragment

<220>
<223> AP39 or ZK248219/248220: labelled derivate of L19

<400> 27
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg

    210                 215                 220

Ile Pro Pro Thr Phe Gly Gln Gly Thr Lys Val Glu Ile Lys Gly Gly
225                 230                 235                 240

Gly Cys Ala

<210> 28
<211> 5
<212> PRT
<213> Artificial Sequence

<220>
<223> Description of Artificial Sequence:antibody or
antibody fragment

<220>
<223> amino acid sequence (bc)

<400> 28
His His His His His
1               5

SEQUENCE LISTING

<110> Bayer Schering Pharma Aktiengesellschaft

<120> ED-B fibronectin as stratification marker for anti-tumor drugs

<130> 41328PEP_US

<140>
<141>

<150>
<151>

<160> 28

<170> PatentIn Ver. 2.1

<210> 1
<211> 238
<212> PRT
<213> Artificial Sequence

<220>
<223> L19 or ZK212667 = single chain variable antibody
      fragment of a monocolonal antibody against the
      extra domain B of fibronectin

<220>

<220>
<223> Description of Artificial Sequence:antibody or
      antibody fragment

<400> 1
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys
225                 230                 235

<210> 2
<211> 4
<212> PRT
<213> Artificial Sequence

<220>
<223> Description of Artificial Sequence:antibody or
      antibody fragment

<220>
<223> amino acid sequence (ba)

<400> 2
Xaa Xaa Xaa Cys
  1

<210> 3
<211> 5
<212> PRT
<213> Artificial Sequence

<220>
<223> Description of Artificial Sequence:antibody or
      antibody fragment

<220>
<223> amino acid sequence (bb)

<400> 3

Xaa Xaa Xaa Cys Xaa
1               5

<210> 4
<211> 4
<212> PRT
<213> Artificial Sequence

<220>
<223> Description of Artificial Sequence:antibody or
      antibody fragment

<220>
<223> amino acid sequence(bc)

<400> 4
His His His His
1

<210> 5
<211> 17
<212> PRT
<213> Artificial Sequence

<220>
<223> Description of Artificial Sequence:antibody or
      antibody fragment

<220>
<223> amino acid sequence (bd)

<400> 5
Ala Ala Ala Asp Asp Asp Ser Asp Asp Asp Tyr Lys Asp Asp Asp Asp
1               5                   10                  15

Lys

<210> 6
<211> 23
<212> PRT
<213> Artificial Sequence

<220>
<223> Description of Artificial Sequence:antibody or
      antibody fragment

<220>
<223> amino acid sequence (bd)

<400> 6

Ala Ala Ala Asp Asp Asp Ser Asp Asp Asp Tyr Lys Asp Asp Asp Asp
1               5                   10                  15

Lys His His His His His His
            20

<210> 7
<211> 121
<212> PRT
<213> Artificial Sequence

<220>
<223> Description of Artificial Sequence:antibody or
      antibody fragment

<220>
<223> amino acid sequence (bd)

<400> 7
Ser Gly Gly Ser Gly Gly Pro Arg Ala Ala Pro Glu Val Tyr Ala Phe
1               5                   10                  15

Ala Thr Pro Glu Trp Pro Gly Ser Arg Asp Lys Arg Thr Leu Ala Cys
            20                  25                  30

Leu Ile Gln Asn Phe Met Pro Glu Asp Ile Ser Val Gln Trp Leu His
        35                  40                  45

Asn Glu Val Gln Leu Pro Asp Ala Arg His Ser Thr Thr Gln Pro Arg
    50                  55                  60

Lys Thr Lys Gly Ser Gly Phe Phe Val Phe Ser Arg Leu Glu Val Thr
65                  70                  75                  80

Arg Ala Glu Trp Glu Gln Lys Asp Glu Phe Ile Cys Arg Ala Val His
                85                  90                  95

Glu Ala Ala Ser Pro Ser Gln Thr Val Gln Arg Ala Val Ser Val Asn
            100                 105                 110

Pro Glu Ser Ser Arg Arg Gly Gly Cys
        115                 120

<210> 8
<211> 5
<212> PRT
<213> Artificial Sequence

<220>
<223> Description of Artificial Sequence:antibody or
      antibody fragment

<220>

<223> HCDR1 = complementarity determining region of the
      region X of the heavy antibody chain

<400> 8
Ser Phe Ser Met Ser
  1               5

<210> 9
<211> 17
<212> PRT
<213> Artificial Sequence

<220>
<223> Description of Artificial Sequence:antibody or
      antibody fragment

<220>
<223> HCDR 2 = complementarity determining region of the
      region X of the heavy antibody chain

<400> 9
Ser Ile Ser Gly Ser Ser Gly Thr Thr Tyr Tyr Ala Asp Ser Val Lys
  1               5                  10                  15

Gly

<210> 10
<211> 7
<212> PRT
<213> Artificial Sequence

<220>
<223> Description of Artificial Sequence:antibody or
      antibody fragment

<220>
<223> HCDR 3 = complementarity determining region of the
      region X of the heavy antibody chain

<400> 10
Pro Phe Pro Tyr Phe Asp Tyr
  1               5

<210> 11
<211> 12
<212> PRT
<213> Artificial Sequence

<220>
<223> Description of Artificial Sequence:antibody or

antibody fragment

<220>
<223> LCDR 1 = complementarity determining region of the
      region X of the light antibody chain

<400> 11
Arg Ala Ser Gln Ser Val Ser Ser Ser Phe Leu Ala
  1               5                  10

<210> 12
<211> 6
<212> PRT
<213> Artificial Sequence

<220>
<223> Description of Artificial Sequence:antibody or
      antibody fragment

<220>
<223> LCDR 2 = complementarity determining region of the
      region X of the light antibody chain

<400> 12
Tyr Ala Ser Ser Arg Ala
  1               5

<210> 13
<211> 10
<212> PRT
<213> Artificial Sequence

<220>
<223> Description of Artificial Sequence:antibody or
      antibody fragment

<220>
<223> LCDR 3 = complementarity determining region of the
      region X of the light antibody chain

<400> 13
Cys Gln Gln Thr Gly Arg Ile Pro Pro Thr
  1               5                  10

<210> 14
<211> 4
<212> PRT
<213> Artificial Sequence

<220>
<223> Description of Artificial Sequence:antibody or

antibody fragment

<220>
<223> preferred amino acid sequence (ba)

<400> 14
Gly Gly Gly Cys
  1

<210> 15
<211> 4
<212> PRT
<213> Artificial Sequence

<220>
<223> Description of Artificial Sequence:antibody or
      antibody fragment

<220>
<223> preferred amino acid sequence (ba)

<400> 15
Gly Cys Gly Cys
  1

<210> 16
<211> 5
<212> PRT
<213> Artificial Sequence

<220>
<223> Description of Artificial Sequence:antibody or
      antibody fragment

<220>
<223> preferred amino acid sequence (bb)

<400> 16
Gly Gly Gly Cys Ala
  1               5

<210> 17
<211> 5
<212> PRT
<213> Artificial Sequence

<220>
<223> Description of Artificial Sequence:antibody or
      antibody fragment

<220>

<223> preferred amino acid sequence (bb)

<400> 17
Gly Cys Gly Cys Ala
  1               5

<210> 18
<211> 6
<212> PRT
<213> Artificial Sequence

<220>
<223> Description of Artificial Sequence:antibody or
      antibody fragment

<220>
<223> preferred amino acid sequence (bc)

<400> 18
His His His His His His
  1               5

<210> 19
<211> 22
<212> PRT
<213> Artificial Sequence

<220>
<223> Description of Artificial Sequence:antibody or
      antibody fragment

<220>
<223> linker amino acid sequence

<400> 19
Met Lys Tyr Leu Leu Pro Thr Ala Ala Ala Gly Leu Leu Leu Leu Ala
  1               5                  10                  15

Ala Gln Pro Ala Met Ala
             20

<210> 20
<211> 242
<212> PRT
<213> Artificial Sequence

<220>
<223> Description of Artificial Sequence:antibody or
      antibody fragment

<220>

<223> AP38: labelled derivate of L19

<400> 20
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Cys

<210> 21
<211> 243
<212> PRT
<213> Artificial Sequence

<220>
<223> Description of Artificial Sequence:antibody or
      antibody fragment

<220>
<223> AP39 or ZK217052/217053: labelled derivate of L19

<400> 21
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Cys Ala

<210> 22
<211> 242
<212> PRT
<213> Artificial Sequence

<220>
<223> Description of Artificial Sequence:antibody or
      antibody fragment

<220>
<223> labelled derivate of L19

<400> 22
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Cys
225                 230                 235                 240

Gly Cys

<210> 23
<211> 243
<212> PRT
<213> Artificial Sequence

<220>
<223> Description of Artificial Sequence:antibody or
      antibody fragment

<220>
<223> labelled derivate of L19

<400> 23
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Cys
225                 230                 235                 240

Gly Cys Ala

<210> 24
<211> 283
<212> PRT
<213> Artificial Sequence

<220>
<223> Description of Artificial Sequence:antibody or
antibody fragment

<220>
<223> ZK225293: labelled derivate of L19

<400> 24
Met Lys Tyr Leu Leu Pro Thr Ala Ala Ala Gly Leu Leu Leu Leu Ala
1               5                   10                  15

Ala Gln Pro Ala Met Ala Glu Val Gln Leu Leu Glu Ser Gly Gly Gly
            20                  25                  30

Leu Val Gln Pro Gly Gly Ser Leu Arg Leu Ser Cys Ala Ala Ser Gly
        35                  40                  45

Phe Thr Phe Ser Ser Phe Ser Met Ser Trp Val Arg Gln Ala Pro Gly
    50                  55                  60

Lys Gly Leu Glu Trp Val Ser Ser Ile Ser Gly Ser Ser Gly Thr Thr
65                  70                  75                  80

Tyr Tyr Ala Asp Ser Val Lys Gly Arg Phe Thr Ile Ser Arg Asp Asn
                85                  90                  95

Ser Lys Asn Thr Leu Tyr Leu Gln Met Asn Ser Leu Arg Ala Glu Asp
            100                 105                 110

Thr Ala Val Tyr Tyr Cys Ala Lys Pro Phe Pro Tyr Phe Asp Tyr Trp
        115                 120                 125

Gly Gln Gly Thr Leu Val Thr Val Ser Ser Gly Asp Gly Ser Ser Gly
    130                 135                 140

Gly Ser Gly Gly Ala Ser Thr Gly Glu Ile Val Leu Thr Gln Ser Pro
145                 150                 155                 160

Gly Thr Leu Ser Leu Ser Pro Gly Glu Arg Ala Thr Leu Ser Cys Arg
                165                 170                 175

Ala Ser Gln Ser Val Ser Ser Ser Phe Leu Ala Trp Tyr Gln Gln Lys
            180                 185                 190

Pro Gly Gln Ala Pro Arg Leu Leu Ile Tyr Tyr Ala Ser Ser Arg Ala
        195                 200                 205

Thr Gly Ile Pro Asp Arg Phe Ser Gly Ser Gly Ser Gly Thr Asp Phe
    210                 215                 220

Thr Leu Thr Ile Ser Arg Leu Glu Pro Glu Asp Phe Ala Val Tyr Tyr
225                 230                 235                 240

Cys Gln Gln Thr Gly Arg Ile Pro Pro Thr Phe Gly Gln Gly Thr Lys
                245                 250                 255

Val Glu Ile Lys Ala Ala Ala Asp Asp Asp Ser Asp Asp Asp Tyr Lys
            260                 265                 270

Asp Asp Asp Asp Lys His His His His His His
        275                 280

<210> 25
<211> 365
<212> PRT
<213> Artificial Sequence

<220>
<223> Description of Artificial Sequence:antibody or
      antibody fragment

<220>
<223> ZK217691/217695: labelled derivate of L19

<400> 25
Met Xaa Asp Thr Pro Ala Glu Val Gln Leu Leu Glu Ser Gly Gly Gly
  1               5                  10                  15

Leu Val Gln Pro Gly Gly Ser Leu Arg Leu Ser Cys Ala Ala Ser Gly
             20                  25                  30

Phe Thr Phe Ser Ser Phe Ser Met Ser Trp Val Arg Gln Ala Pro Gly
         35                  40                  45

Lys Gly Leu Glu Trp Val Ser Ser Ile Ser Gly Ser Ser Gly Thr Thr
     50                  55                  60

Tyr Tyr Ala Asp Ser Val Lys Gly Arg Phe Thr Ile Ser Arg Asp Asn
 65                  70                  75                  80

Ser Lys Asn Thr Leu Tyr Leu Gln Met Asn Ser Leu Arg Ala Glu Asp
                 85                  90                  95

Thr Ala Val Tyr Tyr Cys Ala Lys Pro Phe Pro Tyr Phe Asp Tyr Trp
            100                 105                 110

Gly Gln Gly Thr Leu Val Thr Val Ser Ser Gly Asp Gly Ser Ser Gly
        115                 120                 125

Gly Ser Gly Gly Ala Ser Thr Gly Glu Ile Val Leu Thr Gln Ser Pro
    130                 135                 140

Gly Thr Leu Ser Leu Ser Pro Gly Glu Arg Ala Thr Leu Ser Cys Arg
145                 150                 155                 160

Ala Ser Gln Ser Val Ser Ser Ser Phe Leu Ala Trp Tyr Gln Gln Lys
                165                 170                 175

Pro Gly Gln Ala Pro Arg Leu Leu Ile Tyr Tyr Ala Ser Ser Arg Ala
            180                 185                 190

Thr Gly Ile Pro Asp Arg Phe Ser Gly Ser Gly Ser Gly Thr Asp Phe
        195                 200                 205

Thr Leu Thr Ile Ser Arg Leu Glu Pro Glu Asp Phe Ala Val Tyr Tyr
    210                 215                 220

Cys Gln Gln Thr Gly Arg Ile Pro Pro Thr Phe Gly Gln Gly Thr Lys
225                 230                 235                 240

Val Glu Ile Lys Ser Gly Gly Ser Gly Gly Pro Arg Ala Ala Pro Glu
                245                 250                 255

Val Tyr Ala Phe Ala Thr Pro Glu Trp Pro Gly Ser Arg Asp Lys Arg
            260                 265                 270

Thr Leu Ala Cys Leu Ile Gln Asn Phe Met Pro Glu Asp Ile Ser Val
        275                 280                 285

Gln Trp Leu His Asn Glu Val Gln Leu Pro Asp Ala Arg His Ser Thr
    290                 295                 300

Thr Gln Pro Arg Lys Thr Lys Gly Ser Gly Phe Phe Val Phe Ser Arg
305                 310                 315                 320

Leu Glu Val Thr Arg Ala Glu Trp Glu Gln Lys Asp Glu Phe Ile Cys
                325                 330                 335

Arg Ala Val His Glu Ala Ala Ser Pro Ser Gln Thr Val Gln Arg Ala
            340                 345                 350

Val Ser Val Asn Pro Glu Ser Ser Arg Arg Gly Gly Cys
        355                 360                 365

<210> 26
<211> 244
<212> PRT
<213> Artificial Sequence

<220>
<223> Description of Artificial Sequence:antibody or
      antibody fragment

<220>
<223> ZK210917: labelled derivate of L19

<400> 26
Met Xaa Asp Thr Pro Ala Glu Val Gln Leu Leu Glu Ser Gly Gly Gly
 1               5                  10                  15

Leu Val Gln Pro Gly Gly Ser Leu Arg Leu Ser Cys Ala Ala Ser Gly
            20                  25                  30

Phe Thr Phe Ser Ser Phe Ser Met Ser Trp Val Arg Gln Ala Pro Gly
        35                  40                  45

Lys Gly Leu Glu Trp Val Ser Ser Ile Ser Gly Ser Ser Gly Thr Thr
    50                  55                  60

Tyr Tyr Ala Asp Ser Val Lys Gly Arg Phe Thr Ile Ser Arg Asp Asn
65                  70                  75                  80

Ser Lys Asn Thr Leu Tyr Leu Gln Met Asn Ser Leu Arg Ala Glu Asp
                85                  90                  95

Thr Ala Val Tyr Tyr Cys Ala Lys Pro Phe Pro Tyr Phe Asp Tyr Trp
            100                 105                 110

Gly Gln Gly Thr Leu Val Thr Val Ser Ser Gly Asp Gly Ser Ser Gly
        115                 120                 125

Gly Ser Gly Gly Ala Ser Thr Gly Glu Ile Val Leu Thr Gln Ser Pro
    130                 135                 140

Gly Thr Leu Ser Leu Ser Pro Gly Glu Arg Ala Thr Leu Ser Cys Arg
145                 150                 155                 160

Ala Ser Gln Ser Val Ser Ser Ser Phe Leu Ala Trp Tyr Gln Gln Lys
                165                 170                 175

Pro Gly Gln Ala Pro Arg Leu Leu Ile Tyr Tyr Ala Ser Ser Arg Ala
            180                 185                 190

Thr Gly Ile Pro Asp Arg Phe Ser Gly Ser Gly Ser Gly Thr Asp Phe
        195                 200                 205

Thr Leu Thr Ile Ser Arg Leu Glu Pro Glu Asp Phe Ala Val Tyr Tyr
    210                 215                 220

Cys Gln Gln Thr Gly Arg Ile Pro Pro Thr Phe Gly Gln Gly Thr Lys
225                 230                 235                 240

Val Glu Ile Lys

<210> 27
<211> 243
<212> PRT
<213> Artificial Sequence

<220>
<223> Description of Artificial Sequence:antibody or
      antibody fragment

<220>
<223> AP39 or ZK248219/248220: labelled derivate of L19

<400> 27
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Thr Gly Glu Ile Val Leu Thr Gln Ser Pro Gly Thr Leu Ser Leu Ser
    130                 135                 140

Pro Gly Glu Arg Ala Thr Leu Ser Cys Arg Ala Ser Gln Ser Val Ser
145                 150                 155                 160

Ser Ser Phe Leu Ala Trp Tyr Gln Gln Lys Pro Gly Gln Ala Pro Arg
                165                 170                 175

Leu Leu Ile Tyr Tyr Ala Ser Ser Arg Ala Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Thr Gly Arg
    210                 215                 220

Ile Pro Pro Thr Phe Gly Gln Gly Thr Lys Val Glu Ile Lys Gly Gly
225                 230                 235                 240

Gly Cys Ala

<210> 28
<211> 5
<212> PRT
<213> Artificial Sequence

<220>
<223> Description of Artificial Sequence:antibody or
      antibody fragment

<220>
<223> amino acid sequence (bc)

<400> 28
His His His His His
  1               5